# EUROPEAN PATENT APPLICATION

(11) **EP 1 393 737 A1**
(43) Date of publication of application: **03.03.2004**
(21) Application number: 03255159.0
(22) Date of filing: 20.08.2003
(51) Int. Cl.: A61K 35/78, A61K 35/56, A61P 15/00

(54) **Pharmaceutical compositions and health foods for preventing and treating male sterility comprising oyster extract and ginseng extract**

(30) Priority: 28.08.2002 JP 2002248810
(71) Applicant: TOKIWA KANPO PHARMACEUTICAL CO., LTD., Osaka-shi Osaka-fu (JP)
(72) Inventor: Matsui, Matsutaro, Sakai-shi, Osaka (JP); Kurumiya, Motoshi, Nabari-shi, Mie (JP)
(74) Representative: Jones, Helen Marjorie Meredith

(57) **Abstract**

The present invention provides a pharmaceutical composition and a health food which can be ingested conveniently, and can effectively prevent or treat male sterility. The pharmaceutical composition and the health food comprise an oyster extract and a ginseng extract.

## Description

### FIELD OF THE INVENTION

The present invention relates to a pharmaceutical composition or a health food for preventing or treating male sterility. More specifically, the present invention relates to a pharmaceutical composition or a health food for preventing or treating male sterility comprising an oyster extract and a ginseng extract, which can be ingested conveniently.

### BACKGROUND OF THE INVENTION

Some obstetricians and gynecologists, and urologists report that the frequency of causing a marital sterility derived from male causes (male sterility) is generally 30-50%. Approximately 90% of the causes for the male sterility are associated with sperm and, inter alia, occur by diseases such as oligospermia, asthenozoospermia, azoospermia, and the like. Thus, there is desired development of preventive or therapeutic drugs for the male sterility, which can increase the number of sperms and can be ingested conveniently.

### OBJECTS OF THE INVENTION

An object of the present invention is to provide a pharmaceutical composition which can be ingested conveniently, and can efficiently prevent or treat male sterility, or a health food having the similar effects to those of the pharmaceutical composition.

### SUMMARY OF THE INVENTION

In view of the above object, the present inventors intensively studied and, as a result, found out that a mixture of oyster and ginseng derived from natural products surprisingly exerts superior effects of preventing or treating male sterility, which resulted in completion of the present invention.

Thus, the present invention provides (1) a pharmaceutical composition for preventing or treating male sterility comprising an oyster extract and a ginseng extract as an active ingredient.

Also, the present invention provides (2) a health food for preventing or treating male sterility comprising an oyster extract and a ginseng extract as an active ingredient.

Further, the present invention provides (3) a pharmaceutical composition for increasing the number of sperms comprising an oyster extract and a ginseng extract as an active ingredient.

Also, the present invention provides (4) a pharmaceutical composition for increasing motility of sperms comprising an oyster extract and a ginseng extract as an active ingredient.

Further, the present invention provides (5) a health food for increasing the number of sperms comprising an oyster extract and a ginseng extract as an active ingredient.

Also, the present invention provides (6) a health food for increasing motility of sperms comprising an oyster extract and a ginseng extract as an active ingredient.

### DETAILD DESCRIPTION OF THE INVENTION

The pharmaceutical composition and the health food of the present invention will be explained below.

The pharmaceutical composition and the health food of the present invention contain a mixture of an oyster extract and a ginseng extract as an active ingredient.

An oyster extract used as an active ingredient is obtained from *Crassostreagigas.* Such the extract is obtained by enzymolysis of *Crassostreagigas*.

Upon enzymolysis of *Crassostreagigas*, the following enzymes are used alone or in combination of two or more to treat *Crassostreagigas* in water normally at 40-80°C for 1-10 hours: an acidic protease derived from *Aspergillus niger, Aspergillus oryzae, Aspergillus* sp., *Bacillus* sp., or *Rhizopus niveus*; a neutral protease derived from *Bacillus subtilis, Bacillus* sp., *Aspergillusoryzae, Aspergillus melleus, Pineapple cannery,* or *Carica papaya*; and an alkaline protease derived from *Bacillus subtilis*, or *Bacillus* species. Enzymolysis is performed, for example, in an enzymatic reaction can. By such the treatment, enzymatic hydrolysates and water-soluble components are dissolved in water. Then, the solution is filtrated by using a rotary vaccum filter or the like and is centrifuged at a high speed to give a water-soluble extract, which is filtrated and concentrated with a filter press and a vacuum concentrator to obtain a concentrated extract, which is further dried by freeze-drying and spray-drying to obtain a powder (an oyster extract powder).

The oyster extract powder thus prepared contains large amounts of total amino acid and a free amino acid, such as taurine, proline and arginine.

The oyster extract used in the present invention as an active ingredient also means a concentrate at any step of these concentrating steps. It can be used, for example, as the above-mentioned water-soluble extract, concentrated extract or oyster extract powder for formulating into a pharmaceutical composition or a health food.

The ginseng extract used as an active ingredient in the present invention can be obtained via steps of extracting rhizomes or thin roots of *Panax ginseng* of *Araliaceae* with water, 10-90% hydrous alcohol, or 90-100% ethanol and, then, filtrating, concentrating and drying them.

The ginseng extract may be used at any concentration rate of 1:1-1:100 of the galenical vs. the extract, and it is desired to take 600-30,000 mg as a daily dose of the galenical.

As a raw material for extracting ginseng the materials harvested from any areas such as Siberia, China, Korea, North Korea, America, Japan or Canada can be used, and the raw materials which have been cultivated for one year or more can be used.

After the dried and powdered ginseng is dissolved in no less than 80% ethanol, the procedure of resin adsorption or column separation can give an extract, which is filtrated and concentrated to obtain a dried extract containing a high content of ginseng saponin as a main component.

The content of ginseng saponin in the dried ginseng extract is in a range of 0.2-90.0%, and it is desirable that a daily dose is 600-30,000 mg of the galenical.

The above-resulting oyster extract and ginseng extract can be admixed at a desired ratio to obtain a mixture. It is preferred that a mixing ratio is in a range of oyster extract: ginseng extract = 4,000:5-4,000:600 by weight from a viewpoint of efficacy.

Such the mixture can be formulated into granules and tablets by the known methods, after drying for liquid extracts or without further processing for dried extracts, to obtain a final granule or tablet product in an individual package. Upon formulation into the granules and the tablets, excipients such as lactose, dextrin, starch and cellulose may be employed. Alternatively, such an extract may be filled in a suitable bottle (e.g. glass, can, moisture-proof fiber papers).

A total amount of an oyster extract and a ginseng extract which are incorporated into the pharmaceutical composition of the present invention, is in a range of 200-4,600 mg per one dosage.

It is preferred that in the case of a tablet in which a total amount of an oyster extract and a ginseng extract is 100-400 mg per tablet, 1-20 tablet(s) per one dosage is (are) taken three times a day before breakfast, lunch, and dinner. It is preferred that in the case of a granule in which a total amount of an oyster extract and a ginseng extract is 500-5,000 mg per one bag, 1-4 bag(s) per one dosage is (are) also taken three times a day before breakfast, lunch, and dinner. In addition, it is preferred that in the case of a solution in which a total amount of an oyster extract and a ginseng extract is 0.4-230 mg per 1 ml of preparation, 20-500 ml per one dosage is taken three times a day before breakfast, lunch, and dinner.

In the health food of the present invention, the mixture obtained as described above and the excipient and/or additive as described above can be formulated into a different form of supplemental food (e.g. an individually packaged fine granule, solid pill, and triangular pill), a divided form in which they are redissolved in an aqueous solution to incorporate into a drink, or a divided concentrated solution.

Further, since the oyster extract and the ginseng extract which are active ingredients in the pharmaceutical composition and the health food of the present invention, are components derived from foods, it is not considered that they have toxicity or side effects. Moreover, their mixtures possess the excellent male sterility treating efficacy as shown in the following Examples.

### EXAMPLES

The present invention will be explained in more detail below by way of Preparations and Examples, but is not limited by them.

### PREPARATION 1: Oyster extract

Two thousand kilograms of farm-raised Crassostrea gigas was treated at 60°C for 7.5 hours in water in an enzyme reaction can by using a mixture of a neutral protease derived from *Bacillus* sp. and *Aspergillus oryzae* (Orientase 90N.ONS; Hankyu Bioindustries Inc.) and an acidic protease derived from *Bacillus* sp. and *Aspergillus sp*. (Orientase 20A; Hankyu Bioindustries Inc.), and then filtrated with a vacuum rotary filtration device, centrifuged at a high speed for one hour to obtain the water-soluble extract. The water-soluble extract was concentrated with a filter press and a vacuum rotary filtration device to obtain the concentrated extract. Finally, the concentrated extract was freeze-dried to obtain 120 kg of a power (an oyster extract powder).

### PREPARATION 2: Ginseng extract

One hundred kilograms of ginseng which is bearded roots and out of selection of three years growing roots produced in Kirin Province, China and had been cut into no more than 5 mm pieces, was extracted at 75+/-5°C for 2 hours in 500 kg of a 65% by weight ethanol solution while stirring. The resulting extract solution was subjected to solid-liquid separation to recover a solution. The solution was treated with a filter press to obtain a clear solution. The ethanol in the solution was then evaporated in a concentrator, replaced by water, and the solution was concentrated to no more than 10% of solid content. Since an oil special to ginseng separates out in the upper part of the concentrated solution, the upper solution containing the oil was removed by liquid-liquid-solid separation with a centrifuge. Ginseng saponin was adsorbed onto HP-20 resin (manufactured by Mitsubishi Kasei Corporation) by passing the resulting lower solution through a stainless column charged with HP-20. After adsorption, water-soluble impurities were removed by washing the resin by passing ion-exchange water through the column. The impurities which are poorly soluble in an alcohol were then eluted, and the column was washed by passing ion-exchange water through the column. After washing, the ginseng saponin adsorbed onto the resin was eluted by passing a 70% by weight ethanol solution therethrough, and then concentrated. The resulting concentrated extract was freeze-dried to obtain 1 kg of a powder (containing a ginseng extract and ginseng saponin at 80% by weight).

### EXMAPLE 1

Two hundred milligrams of the resulting oyster extract from PREPARAION 1, 20 mg of the resulting ginseng extract from PREPARATION 2, 20 mg of reducing maltose syrup, 10 mg of dextrin and 10 mg of sucrose fatty acid ester were admixed, granulated, dried, and then sieved at 60 mesh. Following conventional procedures, the pharmaceutical composition of the invention was then obtained in a tablet form (hexagonal tablet, pill or triangular tablet).

### EXAMPLE 2

Three hundred milligrams of the resulting oyster extract from PREPARAION 1, 50 mg of the resulting ginseng extract from PREPARATION 2, 30 mg of reducing maltose syrup, 10 mg of crystalline cellulose and 10 mg of sucrose fatty acid ester were admixed, granulated, dried, and then sieved at 60 mesh. Following conventional procedures, the health food of the invention was then obtained in a tablet form (hexagonal tablet, pill or triangular tablet).

### EXAMPLE 3

Fifteen hundred milligrams of the resulting oyster extract from PREPARAION 1, 150 mg of the resulting ginseng extract from PREPARATION 2, 500 mg of reducing maltose syrup, 150 mg of dextrin and 200 mg of CMC-Ca were admixed, granulated, dried, and then sieved at 10-60 mesh. Following conventional procedures, the mixture was then granulated to obtain the pharmaceutical composition of the invention in a granule form.

### EXAMPLE 4

Fifteen hundred milligrams of the resulting oyster extract from PREPARAION 1, 50 mg of the resulting ginseng extract from PREPARATION 2, 300 mg of reducing maltose syrup, 50 mg of dextrin and 100 mg of CMC-Ca were admixed, granulated, dried, and then sieved at 10-60 mesh. Following conventional procedures, the mixture was then granulated to obtain the health food of the invention in a granule form.

### EXAMPLE 5

Twenty two thousand and five hundred milligrams of the resulting oyster extract from PREPARAION 1, and 180 mg of the resulting ginseng extract from PREPARATION 2 were dissolved in distilled water, to a total amount of 30 ml. The mixture was filled into a 30 ml grass dropper bottle to obtain the pharmaceutical composition of the invention in a solution form.

### EXAMPLE 6

Eighteen thousand milligrams of the resulting oyster extract from PREPARAION 1, and 900 mg of the resulting ginseng extract from PREPARATION 2 were dissolved in distilled water, to a total amount of 30 ml. The mixture was filled into a 30 ml grass dropper bottle to obtain the health food of the invention in a solution form.

### TEST EXAMPLE

### Effect on the number and motility of human sperms.

A test was carried out in human subjects for the effect of improving the number and motility of human sperms and the safety after administration of a mixture of an oyster extract and a ginseng extract.

### 1. Methods

- Test drugs:: (A) Tablet containing 200 mg of oyster extract in one tablet
(B) Tablet containing 200 mg of oyster extract and 20 mg of ginseng extract in one tablet
- Dose:: 10 Tablets/dosage
(2 times per day in the morning and evening)
- Dosing period:: Four weeks
- Number of test subjects:: A total of eighteen male subjects consisting of each 6 male subjects of the following group:
(1) Group of administration of mixture of both oyster and ginseng extracts (including three patients with oligospermia).
(2) Group of administration of oyster extract.
(3) Control group
(No administration of test drugs)
- Measurement periods:: Before administration of test drugs, 2 and 4 weeks after administration of them
- Sampling:: Test subject's sperm samples were obtained by their own masturbation, provided that test subjects did not ejaculate for three days before the sampling to mainly homogenize a density of sperms.
- Measurement:: The number of sperms was counted with a microscope, and their motilities were measured by video recording of sperms via a microscope.
- Criteria:: The number and motility of sperms were compared in each group at 2 and 4 weeks after administration.

### 2. Test results

Changes in the number and motility of sperms with time in each test subject before and after administration of such the test drugs, are shown in Table 1 and 2, respectively.

**Table 1.**

| Effects on the number of human sperms | | | | | | | |
|---|---|---|---|---|---|---|---|
| Groups | Test subject No. | Before administration | | 2 weeks after administration | | 4 weeks after administration | |
| | | ^{a}Number of sperms | Increase rate (%) | Number of sperms | Increase rate (%) | Number of sperms | Increase rate (%) |
| Control (No administration) | 1 | 58 | 100 | 55 | 95 | 56 | 97 |
| | 2 | 55 | 100 | 52 | 95 | 51 | 93 |
| | 3 | 129 | 100 | 109 | 84 | 114 | 88 |
| | 4 | 63 | 100 | 65 | 103 | 61 | 97 |
| | 5 | 66 | 100 | 65 | 98 | 64 | 97 |
| | 6 | 99 | 100 | 85 | 86 | 87 | 88 |
| | Mean | | 100 | 93.5 | | 93.3 | |
| | Standard error | | 0.00 | 7.23 | | 4.41 | |
| Administration of test drug A | 7 | 65 | 100 | 60 | 92 | 66 | 102 |
| | 8 | 73 | 100 | 85 | 116 | 85 | 116 |
| | 9 | 85 | 100 | 90 | 106 | 92 | 108 |
| | 10 | 38 | 100 | 46 | 121 | 53 | 139 |
| | 11 | 103 | 100 | 119 | 116 | 119 | 116 |
| | 12 | 6 | 100 | 7 | 117 | 12 | 200 |
| | Mean | | 100 | 111.3 | | 130.2 | |
| | Standard error | | 0.00 | 10.69 | | 36.44 | |
| Administration of test drug B | 13 | 85 | 100 | 82 | 96 | 95 | 112 |
| | 14 | 79 | 100 | 92 | 116 | 93 | 118 |
| | 15 | 37 | 100 | 46 | 124 | 61 | 165 |
| | 16 | 18 | 100 | 43 | 239 | 67 | 372 |
| | 17 | 87 | 100 | 93 | 107 | 109 | 125 |
| | 18 | 25 | 100 | 56 | 224 | 63 | 252 |
| | Mean | | 100 | 151.0 | | | 190.7 |
| | Standard error | | 0.00 | 63.23 | | | 103.04 |

| | | | | | | | |
|---|---|---|---|---|---|---|---|
| ^{a}Number of sperms (x 10⁶/ml) | | | | | | | |

**Table 2.**

| Effects on motility of human sperms | | | | | | | |
|---|---|---|---|---|---|---|---|
| Groups | Test subject No. | Before administration | | 2 weeks after administration | | 4 weeks after administration | |
| | | Motility of sperms (%) | Increase rate (%) | Motility of sperms (%) | Increase rate (%) | Motility of sperms (%) | Increase rate (%) |
| Control (No administration) | 1 | 55 | 100 | 50 | 91 | 53 | 96 |
| | 2 | 65 | 100 | 63 | 97 | 65 | 100 |
| | 3 | 95 | 100 | 90 | 95 | 85 | 89 |
| | 4 | 55 | 100 | 65 | 118 | 58 | 105 |
| | 5 | 60 | 100 | 55 | 92 | 57 | 95 |
| | 6 | 85 | 100 | 85 | 100 | 81 | 95 |
| | Mean | | 100 | 98.8 | | 96.7 | |
| | Standard error | | 0.00 | 9.95 | | 5.39 | |
| Administration of test drug A | 7 | 55 | 100 | 75 | 136 | 77 | 140 |
| | 8 | 55 | 100 | 65 | 118 | 67 | 122 |
| | 9 | 60 | 100 | 65 | 108 | 70 | 117 |
| | 10 | 50 | 100 | 65 | 130 | 73 | 146 |
| | 11 | 65 | 100 | 80 | 123 | 82 | 126 |
| | 12 | 15 | 100 | 20 | 133 | 30 | 200 |
| | Mean | | 100 | 124.7 | | 141.8 | |
| | Standard error | | 0.00 | 10.50 | | 30.54 | |
| Administration of test drug B | 13 | 79 | 100 | 83 | 105 | 90 | 114 |
| | 14 | 95 | 100 | 98 | 103 | 96 | 101 |
| | 15 | 65 | 100 | 70 | 108 | 85 | 131 |
| | 16 | 75 | 100 | 71 | 95 | 79 | 105 |
| | 17 | 90 | 100 | 95 | 106 | 96 | 107 |
| | 18 | 55 | 100 | 75 | 136 | 89 | 162 |
| | Mean | | 100 | 108.8 | | | 120.0 |
| | Standard error | | 0.00 | 14.05 | | | 23.13 |

While increase in the number of sperms was not recognized in a control group taking no test drugs over a test period, increase in the number of sperms was recognized in a group of administration of a drug, at 2 and 4 weeks after taking them, as compared with before administration. In the oyster extract-administered group, the number of sperms at 2 and 4 weeks after administration of them was increased by 111.3 and 130.2%, respectively and, further, in the mixture of both oyster extract and ginseng extract-administered group, the number of sperms at 2 and 4 weeks after administration of them was remarkably increased by 151.0 and 190.7%, respectively, as compared with before administration (100%). Meanwhile, the motility of sperms was a lower rate in such the mixture-administered group, as compared with the oyster extract-administered group, but increase rates of 108.8 and 120.0% were shown, respectively, at 2 and 4 weeks after administration. Thus, effects of increasing (improving) the number and motility of sperms were recognized in the group in which a mixture of an oyster extract and a ginseng extract was taken, and increase in the number of sperms was more prominent as compared with administration of an oyster extract alone. Abdominal pain, diarrhea, fever, hepatopathy, and other abnormality were not observed in the test subjects taking a test drugs.

Therefore, it was found that such the mixture of the present invention exerts preventive and therapeutic effects useful for male sterility induced from causes such sperm production dysfunction and sperm abnormalities.

### INDUSTRIAL APPLICABILITY

According to the present invention, the pharmaceutical compositions and health foods are provided which can be ingested conveniently, and can exert effective preventive and therapeutic effects on male sterility.

## Claims

1. A composition comprising a combination of an oyster extract and a ginseng extract as active ingredients for preventing or treating male sterility.

2. The composition according to claim 1 which is a pharmaceutical composition.

3. The composition according to claim 1 which is a health food.

4. The composition according to any preceding claim, wherein said oyster extract is obtained from Crassostreagigas.

5. The use of a combination of an oyster extract and a ginseng extract as active ingredients for the manufacture of a composition for the prevention or treatment of male sterility.

6. Use according to claim 5, wherein said composition is a pharmaceutical composition.

7. Use according to claim 5, wherein said composition is a health food.

8. Use according to any of claims 5-7, wherein said composition increases the number of sperm.

9. Use according to any of claims 5-7, wherein said composition increases the motility of sperm.
